# EUROPEAN PATENT SPECIFICATION

(11) **EP 1 968 627 B1**
(45) Date of publication and mention of the grant of the patent: **10.12.2014**
(21) Application number: 06838046.8
(22) Date of filing: 18.11.2006
(51) Int. Cl.: A61K 35/20, A61K 33/38, A61P 17/02, A61K 31/7004, A61K 38/40, A61P 17/10, A61K 33/26, A61K 31/047, A61L 15/32, A61L 15/42, A61L 26/00, A61K 31/05, A61K 9/00, A61K 45/06, A61K 47/26, A61K 9/06, A61K 47/42, A61K 9/12, A61K 9/70, A61L 15/44

(54) **COMPOSITIONS FOR DISRUPTING AND INHIBITING RECONSTITUTION OF WOUND BIOFILM**
ZUSAMMENSETZUNGEN ZUR UNTERBRECHUNG UND UNTERDRÜCKUNG DER REKONSTITUTION EINES WUNDBIOFILMS
COMPOSITIONS POUR L'INTERRUPTION ET L'INHIBITION DE LA RECONSTITUTION DE FILM BIOLOGIQUE DE PLAIES

(30) Priority: 18.11.2005 US 738395 P; 23.06.2006 US 805699 P
(43) Date of publication of application: 17.09.2008
(60) Divisional application: 14182618.0
(73) Proprietor: Glanbia Nutritionals (Ireland) Limited, Kilkenny (IE)
(72) Inventor: WOLCOTT, Randall, Lubbock, Texas 79410 (US)
(74) Representative: O'Brien, John Augustine
(86) International application number: PCT/US2006/044876
(87) International publication number: WO 2007/061942

(56) References cited:
- WO-A1-2004/024180
- WO-A1-2005/018701
- WO-A1-2005/094579
- WO-A2-03/018049
- WO-A2-03/088914
- US-A1- 2004 214 750
- US-A1- 2004 214 750
- US-A1- 2005 053 593
- US-A1- 2005 053 593
- DATABASE WPI Derwent Publications Ltd., London, GB; Class B05, AN 2003-023965, XP008117792 & JP 2002 302404 B (SHISEIDO CO LTD) 18 October 2002
- MASAKO K. ET AL.: 'A novel method to control the balance of skin microflora' JOURNAL OF DERMATOLOGICAL SCIENCE vol. 38, no. 3, June 2005, pages 197 - 205, XP025305662
- MODESTO A. ET AL.: 'Multiple exposures to Chlorhexidine and Xylitol: adhesion and biofilm formation by Streptococcus mutans' CURRENT MICROBIOLOGY vol. 52, no. 6, June 2006, pages 418 - 423, XP019423370
- WEINBERG E.D.: 'Suppression of bacterial biofilm formation by iron limitation' MEDICAL HYPOTHESES vol. 63, no. 5, 2004, pages 863 - 865, XP004602163

## Description

### Cross-Reference to Related Applications

This application claims the benefit of priority of earlier-filed United States provisional patent application number 60/805,699 filed June 23, 2006 and United States provisional patent application number 60/738,395 filed November 18, 2005.

### Field of the Invention

The invention relates to compositions for promoting wound healing. More specifically, the invention relates to compositions for disrupting wound biofilm.

### Background of the Invention

Chronic wounds are an increasingly more significant medical problem. The severity of a medical problem is often reflected in the cost of providing care and, in the U.S. alone, the direct medical costs of chronic wound care are estimated to be several billion dollars. It is estimated that billions of dollars per year are spent on wound care products.

The economic impact of lost productivity resulting from chronic wounds, as both injuries and medical conditions requiring significant care-giver time, is estimated to be about 100 billion dollars. Chronic wounds are frequently associated with a variety of medical conditions that are generally considered to predispose an individual to develop one or more such wounds. For example, according to the American Diabetes Association, there are about 20.8 million diabetics in the United States alone-and that number increases significantly each year. A significant percentage of diabetics develop diabetic ulcers, and 15-25% of individuals with diabetes generally require some type of amputation. More than 60% of the non-traumatic limb amputations occur in people with diabetes, and in 2002 there were about 82,000 non-traumatic lower-limb amputations in the diabetic population in the United States (American Diabetes Association). The prognosis for 5-year survival of a diabetic individual with major limb amputation is 20%, while diabetic non-amputees, even with peripheral vascular disease, have a greater than 60% 5-year survival rate.

Venous leg ulcers affect millions of individuals and millions of individuals have decubitus ulcers.

A vast array of wound care products have been and are being used in an attempt to improve wound healing. Products include antimicrobials, such as compounds containing silver (cadexomer iodine, methyline blue/gentian violet, nonspecific biocides (e.g. hydrogen peroxide, Dakin's solution, and vinegar); topical antibiotics; and moisturizing agents such as hydrocolloid gels, saline compositions (cellulose, alginate, etc.), and medium-chain dextrans (e.g., honey). Wound care products also include systemic and topical anti-infectives, injury management dressings and bandages, wound cleaners, debridement products, silver dressings, moist dressings such as alginates, films, foams, hydrocolloids and hydrogels, biological dressings such as artificial skin collagen and growth factors, and pressure relief products. More recently, biotechnology has contributed a variety of recombinant proteins, peptides, growth factors and other wound therapy products.

WO03/088914A describes inhibiting biofilm formation by contacting bacteria with a metal chelator to thereby limit biofilm formation.

WO2004/024180A describes a lactoferrin composition for treating wounds.

WO03/018049A describes the use of milk serum apoproteins in the prophylaxis or treatment of microbial or viral infection.

WO2005094579A and WO2005018701A describe antimicrobial compositions and methods for reducing biofilm formation.

JP2002302404B describes using a pentose or its sugar alcohol for suppression of bacterial biofilms.

Journal of Dermatological Science vol. 38, issue 3, pages 197-205 (2005) describes the effect of xylitol and farnesol in inhibiting biofilm formation caused by Staphylococcus aureus*.*

Current Microbiology, 2006, vol. 52, No. 6, pages 418-423 describes the effect of chlorhexidine and xylitol on biofilm formation caused by Streptococcus mutans*.*

Medical Hypotheses, vol. 63, Issue 5, pages 863-865 [2004] describes inhibition of bacterial biofilm formation by iron limitation.

US20050053593A describes an antimicrobial composition comprising an antimicrobial lipid and an enhancer component.

US2004/0214750A describes the treatment of dermatological conditions by administering lactoferrin topically to the skin and in the same timeframe administering lactoferrin orally.

Although hundreds of different wound care compositions have been used in an attempt to improve wound care, according to the United States Centers for Disease Control, in 2002 the age-adjusted lower-extremity amputation rate (2.9 per 10,000 population) was almost twice that of the rate in 1980 (1.6 per 10,000 population). Clearly, a great need still exists for better products and methods for wound management and therapy.

### Summary of the Invention

According to one aspect the invention provides a wound care composition for use in the treatment of biofilm-associated wounds as defined in the claims.

According to another aspect the invention provides a wound care composition for use in increasing the rate of healing of a biofilm-associated wound as defined in the claims.

Also provided is a wound dressing as defined in the claims.

Also described are methods for providing wound treatment. In one case, the method comprises applying to an acute or chronic wound a composition comprising MDPP, or isolated lactoferrin, and xylitol. In another case, the method may also comprise applying to the wound at least one moisturizing agent such as, for example, methylcellulose/gelatin gel. Also described are methods for treating chronic wounds by applying to a debrided wound a composition comprising an inhibitor of biofilm reconstitution (IBR) which, in various embodiments, may comprise lactoferrin and xylitol, MDPP and xylitol, MDPP, MDPP-A, or MDPP-A and xylitol. Also described is a method for inhibiting reconstitution of biofilm from biofilm fragments in a human or animal tissue is also provided, the method comprising applying to the tissue a bovine milk-derived protein product processed to provide an enriched concentration of from about 2% to about 100% of the milk-derived protein product as lactoferrin and from about 0 to about 15 mg Fe/100g lactoferrin.

### Brief Description of the Drawings

Fig. 1 is a photograph of a chronic wound, illustrating the presence of biofilm in the wound as evidenced by the substantial quantity of visible slough.
Fig. 2a is a photograph of a chronic wound before treatment, and Fig. 2b is a photograph of the same wound after treatment with a MDPP/xylitol composition as provided in one embodiment of the invention.
Fig. 3a is a photograph of a chronic wound before treatment, and Fig. 3b is a photograph of the same wound after treatment with a MDPP/xylitol composition as provided in one embodiment of the invention.

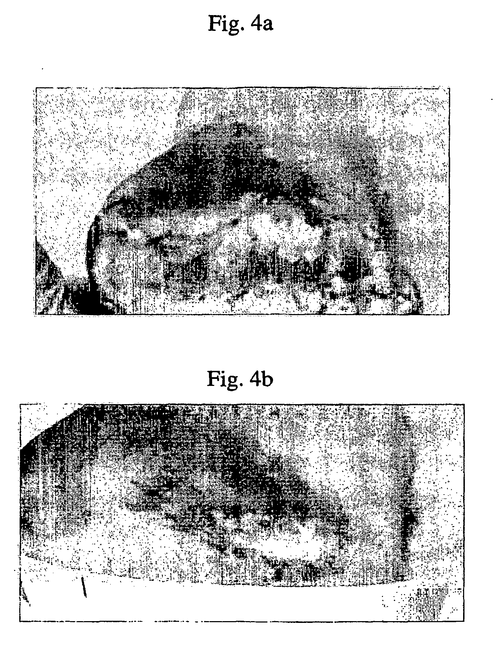

Fig. 4a is a photograph of a chronic wound before treatment, and Fig. 4b is a photograph of the same wound after treatment with a MDPP/xylitol composition.
Fig. 5a is a photograph of a chronic wound before treatment, and Fig. 5b is a photograph of the same wound after treatment with a MDPP/xylitol composition.
Fig. 6a is a photograph of a chronic wound before treatment, and Fig. 6b is a photograph of the same wound after treatment with a MDPP/xylitol composition. In this case, the composition was combined in an Aquaphor® (Smith and Nephew Wound Care, Hull, UK) base.

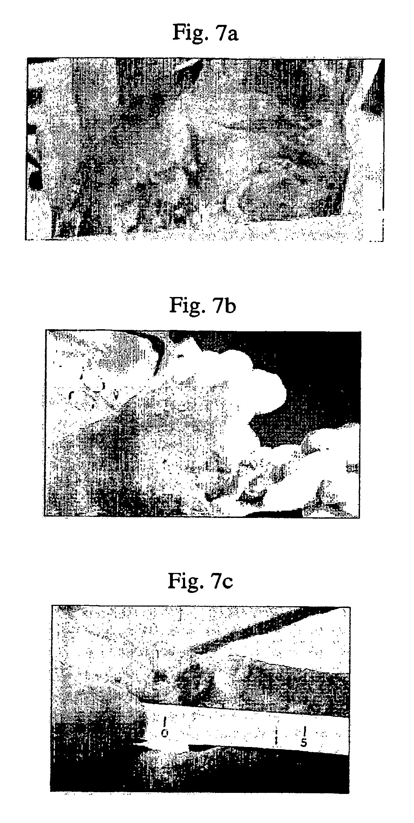

Fig. 7a is a photograph of a diabetis foot ulcer with osteomyelitis of the left toe. Initial hospital recommendation was that the left foot be amputated. Figs. 7b and 7c were taken after initiation of treatment at the Southwest Regional Wound Care Center, Lubbock, Texas. Fig. 7b is a photograph of a debrided wound of the left foot, resulting in significant tissue loss to the great toe. Fig. 7c is a photograph of the same wound less than 3 months later. The wound was treated with a MDPP/xylitol gel.

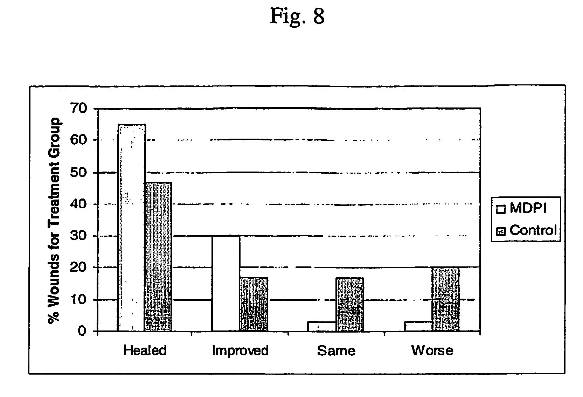

Fig. 8 is a graph illustrating the effects of treatment of 67 wounds (37 with milk-derived protein product and 30 with Curasol® methylcellulose gel as control) over an 8-week period. A variety of types of wounds were included in the study, including venous leg ulcers, traumatic wounds, non-healing surgical wounds, diabetic foot ulcers, wounds in patients with venous insufficiency, and decubitus ulcers. Wound margins were traced and wound volume, expressed in cubic centimeters, was calculated weekly. Wounds were categorized, based upon wound characteristics and volume change of wound, as: healed, improved, same, or worse. Results are illustrated in the graph with the wound category on the X axis and the percentage of wounds in the treatment group belonging to each category shown on the Y axis. As illustrated by the graph, MDPP treatment resulted in significantly more healed and improved wounds than did standard hydrogel treatment.

### Detailed Description

The inventor has discovered that a composition comprising bovine milk-derived protein product (MDPP) provides a safe, highly-effective, and affordable topical wound care agent. As used herein, MDPP refers to a milk-derived fraction from a non-human mammal that contains an enriched concentration (*i.e.,* greater than 2%) of lactoferrin. Compositions of the present invention that the inventor has found to be especially effective for promoting wound healing comprise MDPP and xylitol. Such compositions may also comprise isolated mammalian lactoferrin (including human lactoferrin), or subunits or variants thereof, in combination with xylitol. Xylitol is a polyol, and a 5-carbon open chain sugar alcohol having the chemical name 1,2,3,4,5-Pentahydroxypentane. Pure xylitol is a white crystalline substance that looks and tastes like sugar. Natural sources of xylitol include, for example, plums, strawberries, and raspberries. While not being bound by theory, it is believed that the activity of xylitol may lie in its absence of reducing carbonyl groups. Therefore, non-reducing sugar alcohols such as, for example, sorbitol may also provide a benefit when used in a composition such as those described. Lactoferrin (LF) is an iron-binding glycoprotein generally consisting of a single polypeptide chain with a molecular weight of 75,000 - 80,000 Daltons. LF can be isolated from a wide variety of dairy products and dairy- derived ingredients using industrial scale chromatography. Compositions of the present invention may comprise a milk-derived protein product as described herein, but may also optionally utilize isolated or purified lactoferrin such as, for example, bovine milk-derived lactoferrin, other mammalian milk-derived lactoferrin, recombinant human lactoferrin, or variants of recombinant mammalian lactoferrin, in combination with xylitol to provide wound healing compositions. It is also within the scope of the invention to provide other non-human mammalian milk-derived protein products comprising at least about 2 to about 99 percent lactoferrin by weight in compositions for wound healing.

The inventor has discovered that MDPP or isolated lactoferrin, in combination with xylitol, is especially effective for the treatment of chronic, non-healing wounds. A chronic wound is defined herein as a wound that fails to progress through an orderly and timely sequence of repair or a wound that does not respond to treatment and/or the demands of treatment are beyond the patient's physical health, tolerance or stamina. Those of skill in the art of wound care understand that many wounds that are, at first, considered to be acute wounds ultimately become chronic wounds due to factors still not well understood. The inventor has determined that one very significant factor is the transition of planktonic bacteria within the wound to form a biofilm. Biofilm disruption, or inhibition of biofilm reconstitution, as used herein refers to the property demonstrated by the components of the present invention related to their ability to clear biofilm from a chronic or biofilm-containing acute wound or inhibit reconstitution of a biofilm mass from the remnants remaining after debridement and thereby promote healing of the wound.

In one aspect, the MDPP comprises at least about 10% bovine lactoferrin, which can have an iron content of from about 15mg/100g to about 40mg/100g. In other aspects, the MDPP may comprise at least about 50%, at least about 75%, or at least about 90% lactoferrin. One form of a milk-derived protein product may also comprise the apolactoferrin form of lactoferrin, providing an iron concentration of from about 0mg/100g to about 15mg/100g ("MDPP-A"). A composition comprising bovine MDPP or MDPP-A, or optionally an isolated mammalian lactoferrin protein such as bovine or human lactoferrin, or a variant thereof, in combination with xylitol is particularly effective for wounds that have been resistant to conventional wound-healing methods-wounds that might, under other circumstances, qualify an individual for amputation of the tissue surrounding the wound site. Aspects of the invention also provide compositions comprising MDPP or MDPP-A, xylitol, and at least one pharmaceutically acceptable carrier suitable for topical administration of the composition to the skin or to a wound surface. Bioferrin^{®} 1000 (Glanbia Nutritionals, Inc., Monroe, Wisconsin) is a natural, biologically-active MDPP-A. Bioferrin^{®} 2000 (Glanbia Nutritionals, Inc.) is a MDPP having higher iron content (15-40 mg/100g). Xylitol may be obtained from a variety of commercial vendors such as, for example, Spectrum Chemicals and Laboratory Products, Gardena, California USA. Xylitol may also be isolated from natural sources including, for example, plums, strawberries, and raspberries.

Milk-derived protein isolates containing lactoferrin typically contain from about 15 to about 45 mg iron per 100 grams of protein isolate, but may also contain as much as 60 mg per grams of protein isolate. The inventor has discovered that a milk-derived protein isolate processed to provide from about 0 to about 15 mg iron per 100 grams of protein isolate (*i.e.,* lactoferrin is provided in the form of apolactoferrin) provides an especially effective wound healing agent when topically applied to the wound. While lactoferrin inhibits reconstitution of an existing biofilm in a chronic wound, this processed MDPP-A is even more effective at doing so-and a combination of lactoferrin and xylitol, especially apolactoferrin and xylitol, is very effective at inhibiting reformation of a more extensive biofilm from the biofilm remnants remaining after debridement of a chronic wound. The inventor has also discovered that a subset of individuals with chronic wounds experience an uncomfortable burning sensation upon application of MDPP, but this can be avoided by using MDPP-A. MDPP-A may also be used at lower concentrations, while still providing an increased rate of healing and number of healed wounds over that of MDPP and over that of currently available wound care products. Bioferrin^{®} 1000 (Glanbia Nutritionals, Inc.) is a natural, biologically-active milk-derived protein product (MDPP-A) comprising bovine apolactoferrin from fresh sweet whey. It is isolated using fractionation separation techniques known to those of skill in the art and comprises greater than 90 percent protein, with greater than 90% of the total protein (e.g., 95%) comprising lactoferrin, primarily in the apolactoferrin form. Bioferrin^{®} 1000 also has a moisture content of less than approximately 5 percent and less than approximately 2 percent ash. It is provided as a dry powder having a pH of greater than approximately 6.0 (1% solution at 20° C), and may therefore be applied to a wound as a powder or admixed into a gel, cream, liquid, or other suitable pharmaceutical base for topical application to a wound. It may also readily be mixed with xylitol to form a wound-healing composition that may be applied as a powder, cream, gel, liquid, aerosol, or other topical preparation.

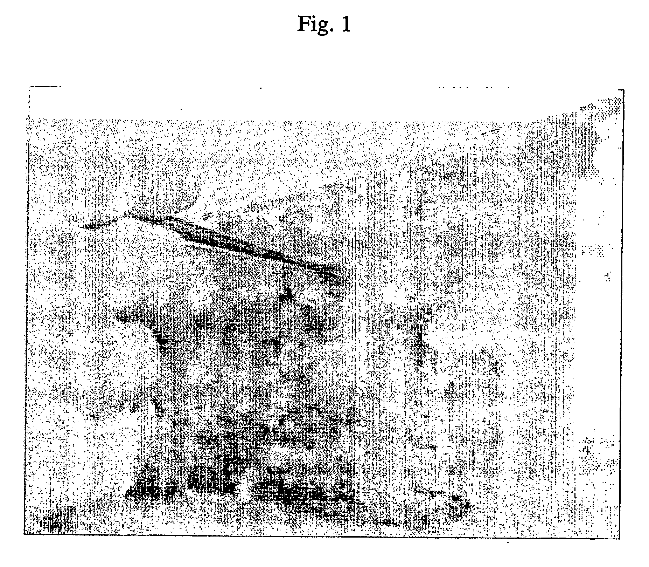

Slough, which can be seen on the surface of a wound as in Fig. 1, is comprised predominantly of mixed-species bacterial biofilm, according to an analysis of wound slough samples performed for the inventor by the Center for Biofilm Engineering at Montana State University. Chronic wounds have surprising similarities. At a biochemical level, chronic wounds display increased proinflammatory cytokines, increased matrix metalloproteases in a specific pattern, low levels of tissue inhibitors of matrix metalloproteases, low levels of growth factor cytokines along with degraded receptors on the cells constituting the wound bed (senescent cells). The presence of biofilm, which is ubiquitous in chronic wounds and a common characteristic of non-healing wounds, is consistent with each of the similarities found in chronic wounds. Biofilms demonstrate remarkable colony defenses against antibiotics, biocides, human immune system defenses, and most of the current wound care products used to treat chronic wounds today.

Biofilms isolated from any individual wound may comprise multiple different types of bacteria, both Gram positive and Gram negative. Whether among those with readily detectable biofilm or not, however, wounds are generally populated by a variety of bacterial species as well as fungal species. Furthermore, bacteria are more difficult to isolate and culture when present in the form of a biofilm, in part because they may be removed by swab or debridement as clusters of cells comprising multicellular matrix-enclosed biofilm fragments that do not form colonies when plated for identification and quantification. (Costerton, W., et al., J. Clin. Invest. (2003) 112(10) 1466-1477.)

In one study, for example, study participants were chosen based upon the diagnosis of presence of non-infected wounds, the diagnosis having been made because of the lack of clinical signs of infection. Only 14.3% of those wounds in the control group and only 40.7% of wounds in the ultrasound treatment group were healed in 11-12 weeks, and investigators discovered that the "uninfected wounds" of more than 86% of the study participants contained greater than 100,000 colony-forming units of bacteria per gram of tissue. What was especially noteworthy was that the samples were cultured after debridement, suggesting that bacterial biofilm are not currently effectively identified as an inhibitor of healing, resulting in ineffective treatment and poor clinical outcomes (Ennis, W. *et al* (2005) 51 (8): 24-39).

Rayner *et al.* demonstrated that a whey extract comprising growth factors could stimulate healing of an incisional wound in an animal model. (Rayner, T.E. et al., Am. J. Physiol. Regulatory Integrative Comp Physiol. (2000) 278: 1651-1660.) As Rayner *et al.* point out, however, growth factors that have been shown to promote wound repair in experimental animals have not generally demonstrated the same effects in clinical studies of chronic wound healing. Earlier studies had shown that bovine lactoferrin was not effective for treating oral ulcers in hamsters (Clarke, J. et al., Oral Oncol. (1999) 35(2): 197-202), indicating that bovine lactoferrin, although considered by some as a good antimicrobial, was not effective for wound healing. The inventor has demonstrated that a bovine milk-derived protein product comprising lactoferrin is very effective for the treatment of acute and chronic wounds that would otherwise exhibit delayed healing due to the development of biofilm in the wound space and the difficulty usually experienced in clearing an established biofilm from a wound.

*In vitro* studies have demonstrated that the iron-sequestering properties of lactoferrin, a protein found in bovine milk and whey products, stimulate *P*. *aeruginosa* surface motility and block biofilm formation. (Singh, P.K. BioMetals (2004) 17: 267-270.) Biofilm formation, however, involves the transition from planktonic cell phenotype to biofilm cell phenotype, and the phenotypes have been shown to be vastly different (Resch, A. et al., Appl. Env. Micro. (2005) 71(5): 2663-2676). The pure cultures often used to study biofilm formation have therefore been suggested to be a very poor model for the study of biofilms and the organisms that form them (Costerton, W. et al., J. Clin. Invest. (2003) 112 (10): 1466-1477). *Pseudomonas aeruginosa* isolated from a burn wound has been shown to develop a biofilm within a few hours (Harrison-Balestra, C. et al., Dermatol. Surg. (2003) 29: 631-635), and chronic wounds have been colonized by biofilms for weeks, months, or even years. It is therefore more difficult to determine from an *in vitro* demonstration of inhibition of biofilm formation whether any particular agent will be effective in a wound comprising an established biofilm. Furthermore, while an agent such as lactoferrin has demonstrated the ability to block initial biofilm formation by *Pseudomonas in vitro,* lactoferrin and iron depletion were actually demonstrated by Francesca *et al.* to stimulate biofilm formation by certain bacterial species, such as the Gram positive microorganism *Streptococcus mutans.* (Francesca, B. et al., BioMetals (2004) 17: 271-278.) Johnson *et al.* also demonstrated that *Staphylococcus aureus* biofilm production is induced in low-iron conditions and repressed by iron. Even more puzzling have been studies indicating that elevated iron concentrations produced by application of various iron salts to bacterial cultures in 384-well plates (Musk, D. et al, Chem. Biol. (2005) 12: 789-796) appear to inhibit biofilm formation. Furthermore, studies by Arnold et al (Infect. Immun. (1982) 35(3): 792-799) indicated that access to the bacterial cell surface, which would be complicated by the matrix surrounding bacteria in a biofilm, is essential for the microbicidal effects of lactoferrin that were previously demonstrated on non-lactoferrin-resistant planktonic bacteria. Bacteria encase themselves in a polysaccharide and protein matrix within a biofilm, where they are generally considered to be resistant to antibiotic administration (Stewart, P. and J. Costerton, Lancet (2001) 358: 135-138).

The disparities between observations made for individual agents and their effects on bacterial populations, *in vitro* wound models, etc., combined with the complexity of factors contributing to the wound environment have made it considerably more difficult to identify effective wound care compositions, particularly for chronic wounds that have established biofilm masses within the wound and have demonstrated resistance to antibiotics and other wound therapy agents.

The inventor has demonstrated the efficacy of compositions of the present invention *in vivo* in a variety of human chronic/non-healing wounds in a significant number of patients. Compositions in accordance with aspects of the present invention have demonstrated effectiveness *in vivo* in a clinical setting, and among the wounds healed as the result of treatment with these compositions have been many wounds formerly thought to be resistant to healing. For example, in multiple cases of diabetic foot ulcer where the composition has demonstrated effectiveness in healing a wound, the wound was serious enough to warrant classification as a Wagner's grade 4--an ulcer that has led to gangrene of the toes and/or forefoot.

Reconstitution of existing biofilm following debridement is a significant factor in the non-healing behavior of chronic wounds. Antibiotic/antiseptic therapies are ineffective against an existing biofilm, since the bacteria in the biofilm have already transitioned from the planktonic phenotype to the phenotype of a member of a biofilm community and the compositions comprising a biofilm protect the microorganisms within it from compounds that would generally kill a planktonic cell. Removal of slough by debridement is helpful, as it results in removal of a significant amount of biofilm from the wound. However, biofilm is difficult, if not impossible, to remove in its entirety by standard debridement techniques, and biofilm tends to rebuild itself from remnants remaining in and around the wound space to return to or near its original volume, or reconstitute, quickly after debridement and standard therapy. Agents effective at inhibiting biofilm reconstitution are especially effective at promoting healing in a wound that was previously considered to be "non-healing."

Based on clinical results, a combination of milk-derived protein product (MDPP), preferably comprising at least about 10% bovine lactoferrin, in conjunction with xylitol, provides an effective and well-tolerated composition for inhibiting reconstitution of an established biofilm and promoting wound healing when applied topically to a wound. When a milk-derived protein product (MDPP) comprising Bioferrin® 2000 (Glanbia Nutritionals USA, Monroe, Wisconsin) was utilized, a significant percentage of wounds responded to the application, and later results demonstrated that when MDPP-A was used in combination with xylitol in a topically-applied wound care composition, the combination additionally increased the percentage of wounds that responded to topical application of the wound care composition. More recently, the inventor has discovered that wounds treated with MDPP-A, provided in the clinic as Bioferrin^{®} 1000, progressed more rapidly to healing than did wounds treated with MDPP, although results were good with MDPP.

In one embodiment, compositions are provided comprising MDPP (or, more preferably, MDPP-A), xylitol and at least one moisturizing agent, such as, for example, a hydrocolloid gel, a saline composition, a medium-chain dextran (e.g., honey), etc., for application to an acute or chronic wound. Commercially available compositions such as, for example, DuoDerm® Hydroactive Dressings (Bristol-Myers Squibb, Princeton, NJ), can be used as a base into which a suitable amount of MDPP (Bioferrin®, Glanbia Nutritionals, Inc., Monroe, WI) is admixed. Moisturizing agents suitable for wound care are known to those of skill in the art, and a number of such agents are commercially available. In another embodiment, compositions are provided comprising MDPP or MDPP-A, xylitol, and a silver product such as Acticoat^{®} (Smith and Nephew, Memphis, Tennessee).

Compositions of the present invention may readily be used in conjunction with compositions and devices containing antimicrobial preparations of silver. Acticoat^{®}, for example, may provide antimicrobial silver as a rayon/polyester non-woven core laminated between an upper and lower layer of silver-coated high density polyethylene (HDPE) mesh. Acticoat^{®} Moisture Control (Smith and Nephew, Memphis, Tennessee) is a foam dressing comprising silver in nanocrystalline form. Actisorb^{®} Silver products distributed by Johnson and Johnson comprise activated charcoal cloth impregnated with silver (33 µg silver per square cm of cloth). Arglaes^{®} products (Giltech Ltd., UK) also provide polymers for release of silver ions from a powder, film dressing, or other preparation for application to a wound. Compositions of the present invention may be incorporated into such products or used in combination with such products. One advantage of augmenting the use of silver products with the use of compositions described by the invention is the ability to incorporate less silver into the products while achieving an improved effect on wound healing. As used herein in regard to silver-containing wound dressings, both compositions and devices are included, and may include woven or non-woven fabrics or polymers, alginates, foams, powders, gels, creams, liquids, wound fillers and other pharmaceuticals or medical devices deemed by those of skill in the art to be appropriate for effective delivery of silver compositions to a wound.

The composition is preferably applied following wound debridement. Although biofilm bacteria cannot be completely eradicated from the wound area by debridement, decreasing biofilm mass and providing increased exposure of the debrided tissue and remaining biofilm bacteria to the inventive compositions increases wound healing. The slough that fills a chronic wound, previously thought to be comprised of dead cells, cellular debris, bacteria, and tissue fluid, has recently been demonstrated to be comprised primarily of a mixed-species bacterial biofilm. It is therefore of benefit to debride the slough from the wound as completely as possible. Debridement can be performed by surgical, mechanical, autolytic, enzymatic, or a combination of means known to those of skill in the art of wound care. Furthermore, standard gauze dressings to pack or cover the wound will preferably be avoided, since those dressings may enhance biofilm formation or reconstitution in the wound. If used, such dressings may, however, be coated or impregnated with a composition of the present invention to decrease the biofilm-enhancing properties of a gauze dressing.

One suggested course of treatment, for example, comprises weekly debridement of a slough-containing wound and application of a composition as a fresh bandage is applied, which may be three times per week.

Topical wound care compositions may comprise about 2 to about 200 mg of MDPP or MDPP-A per cubic centimeter of total composition. In one embodiment, a composition comprises about 20 to about 200 mg of MDPP or MDPP-A, provided as Bioferrin^{®}1000 or Bioferrin^{®}2000, per cubic centimeter of total composition and about 1 to about 50, or in some embodiments about 5 to about 20, percent (w/v) xylitol. In one embodiment, this composition may be admixed into a methylcellulose/gelatin gel. This composition can be applied, in an amount of about ½ to about 5 cc of protein isolate/xylitol composition to each one square centimeter of wound area, to a freshly debrided chronic wound after the slough has been removed.

To maintain a thicker consistency prior to application the composition may be stored at about 4°C. The composition may also be combined with a petrolatum base, such as Aquaphor® (a 41% w/w petrolatum base available from Smith and Nephew Wound Care, Hull, UK), to maintain a consistency that will improve adherence of the composition to certain wounds, if needed. Compositions of the invention may also comprise powders, solutions, ointments, aerosol sprays, and/or creams.

Traditionally, a wet-to-dry dressing (e.g., gauze wetted with normal saline) has been the standard dressing used by up to 80% of the physicians in the United States. Studies have shown that using this type of dressing results in healing in about 40-45% of wounds. Some use more recently-developed dressings of hydrogel and a semi-permeable primary dressing, and this has resulted in an increase in the percentage of wound healing to about 70%. The inventive compositions and methods, however, have been applied to hundreds of wounds to achieve minimally an 85% to 90% rate of wound healing in chronic wounds. The compositions and methods have also demonstrated efficacy in healing wounds that often would, under other circumstances, qualify a patient for amputation of the affected area.

Furthermore, the compositions have demonstrated efficacy in protecting skin tears and other acute wounds in elderly patients in nursing homes from advancing to more serious wounds. These patients tend to be immunocompromised and more likely to develop chronic wounds. They also live in an environment in which they are more likely to be exposed to a variety of bacteria, often already in the biofilm state, and their acute wounds may quickly progress to the biofilm-infected chronic state. Therefore, compositions are provided comprising MDPP and/or MDPP-A, as well as compositions comprising MDPP and/or MPDI-A and xylitol, for the treatment of acute wounds such as, for example, cuts, cuts, scrapes, and tears of the skin. The compositions have been effectively used with nursing home patients who frequently experience skin tears, where they have been observed to decrease the frequency of transition from acute skin wound to chronic wound. Compositions may be applied topically to a wound as powders, aerosols, creams, gels, or other topical preparations or they may be applied to a bandage prior to application of the bandage to the wound. Bandage surfaces may be coated with compositions of the invention, or the bandaging material may be impregnated with compositions of the invention.

By treating individuals with chronic wounds with the compositions, the inventor and his colleagues at the Southwest Regional Wound Care Center have demonstrated that an inventive wound care composition can promote healing of the types of wounds that would previously have been the basis for a decision to amputate the limb on which the wound was located.

Methods for treating chronic wounds are also described, the methods comprising applying to a chronic wound a composition comprising an agent that disrupts biofilm reconstitution or reattachment. Biofilm formation plays a key role in the transition from acute wound to chronic wound, and currently used antibiotic regimens, biocides, dressings, etc. are significantly less effective because of the presence of biofilm in the wound space. Following debridement to remove the slough that consists primarily of biofilm, application of a composition comprising an agent that disrupts biofilm reconstitution, in certain aspects in combination with a moisturizing agent, promotes wound healing in a manner that significantly increases the percentage of healing in chronic, previously non-healing wounds. Biofilm reconstitution can generally be detected by the reformation of slough in the wound space, and this slough is significantly reduced or eliminated as a result of treatment with compositions of the invention. In wounds in which a significant mass of biofilm is not visible to the human eye, compositions of the invention may be applied without first debriding the wound.

In one embodiment, compositions comprising MDPP, MDPP-A, lactoferrin, MDPP/xylitol, MDPP-A/xylitol, or lactoferrin/xylitol and at least one moisturizing agent, such as, for example, a hydrocolloid gel, a saline composition, a medium-chain dextran (*e.g*., honey), etc., are provided for application to a chronic wound to inhibit biofilm reconstitution following debridement. A moisturizing agent, as used herein, is an agent that promotes retention of water in the wound bed, such as an occlusive dressing, or is an agent that can donate water to the wound bed, such as a hydrogel. Matrices that absorb aqueous solutions and/or water and release it into the wound bed over a period of time are often especially effective as moisturizing agents for wound care. Commercially available compositions such as DuoDerm® Hydroactive Dressings (Bristol-Myers Squibb, Princeton, NJ), for example, can be used as a base into which a suitable amount of MDPP or MDPP-A (*e.g*. Bioferrin®1000 or Bioferrin®2000, Glanbia Nutritionals, Inc., Monroe, WI) is admixed. Moisturizing agents suitable for wound care are known to those of skill in the art, and a number of such agents are commercially available. In certain aspects, the composition is applied following wound debridement.

The inventor has discovered that biofilm is present in a variety of wounds, that it forms quickly in the wound space, and that it is primarily responsible for the chronic, non-healing nature of wounds commonly referred to as "chronic" wounds, as well as increased time to healing for "non-chronic" wounds. Much study has been done in the area of inhibiting biofilm formation on a surface, but by the time most patients present to a clinic or hospital, or by the time they realize themselves that a wound is serious enough to warrant the application of topical agents to improve healing, biofilm has already formed in the wound space. This biofilm consists of cells of altered phenotype as compared to the planktonic cells from which they formed. Therefore, the inventor has focused his attention on identifying agents that act as inhibitors of biofilm reconstitution-those agents that disrupt biofilm or inhibit it from reforming from the remnants that remain in the wound area after debridement.

The inventor has discovered that bovine milk protein products comprising at least about 2% lactoferrin by weight are effective agents for disrupting biofilm reformation or reconstitution. A particularly effective composition for this purpose is a composition comprising a combination of lactoferrin and xylitol, optionally comprising a moisturizing agent that may be used to form a base for the composition. The composition may be admixed into a methylcellulose gelatin gel. This composition can be applied in an amount of about ½ to about 5 cc of lactoferrin/xylitol composition to each one square centimeter of wound area to a freshly debrided chronic wound after the slough (biofilm) has been removed.

A composition may also be applied to a wound by use of a hydrophilic superabsorbent wound dressing, such as that described in United States Patent number 6,399,092 (Hobson, et al. 2002). A preparation using hydrophilic dextran polymer beads may also be used for wound treatment, the preparation comprising hydrophilic dextran polymer beads and MDPP, MDPP-A, lactoferrin and xylitol, MDPP and xylitol, MDPP-A and xylitol, or combinations thereof. Preparation of cross-linked beads for releasing an active agent into the wound while absorbing wound exudate are described in U.S. Patent Number 4,783,448 (Johansson, 1988). Compositions of the invention may also be applied to wound dressings, bandages, wound fillers, and other agents used for wound care to deliver the active agents to the wound or to provide additional benefit by making the bandage, filler, or other material less conducive to the development of a biofilm on the wound care dressing or bandage. Compositions may be combined with, and/or applied to, a variety of different types of wound care products, including but not limited to hydrogel sheets, hydrogel wound fillers, absorptive dressings, alginates, compression dressings and wraps, composite dressings, and transparent films.

One advantage of a wound care composition comprising MDPP, MDPP-A, lactoferrin, lactoferrin/xylitol, MDPP/xylitol, MDPP-A/xylitol, or combinations thereof lies in the safety of these ingredients if consumed. Wound care products, especially when applied to the surface of a large wound, may be absorbed by the tissue and distributed throughout the body. This can be a concern if components of the wound care products, such as iodine or silver nitrate, may have toxic effects if absorbed to a significant degree. Milk-derived lactoferrin, however, is generally recognized as safe at levels of consumption of 100 mg/product serving or 1.0 g/person/day (U.S. Food & Drug Administration, GRN000077 and GRN0000130).

Traditional wound care has distinguished between different types of chronic wounds, but the present compositions can be used for any chronic wound, including, for example, diabetic foot ulcers, decubitus ulcers, venous leg ulcers, and postoperative surgical wounds. The compositions may also be used for biofilm-based infections which are accessible for application of MDPP, MDPP-A, MDPP/xylitol, MDPP-A/xylitol, or lactoferrin and xylitol, and which are likely to comprise mixed populations of bacterial species. These infections include, for example, sinus infections, rectal infections, oral lesions, intestinal conditions associated with biofilm or increased (*i.e.,* augmented) biofilm formation, and infections associated with indwelling devices such as catheters.

Acne vulgaris is a common skin disorder that affects a significant percentage of the population, especially during adolescence. It is often associated with the bacterium *Propionibacterium acnes,* and has been associated with biofilm formation. Compositions in accordance with embodiments of the present invention provide a benefit for topical application for the treatment of such a biofilm-associated condition, particularly when provided in association with standard therapies such as systemic antibiotic treatment. Substances such as pluronic lecithin organogels (PLOs) may be used to provide the moisturizing base and promote absorption of the active agents into the skin.

The scientific and medical communities continue to identify diseases and disease states that are associated with the presence of biofilm, or augmented biofilm, in tissues. Augmented biofilm is generally an increased presence of biofilm or the presence of a significant number of bacteria in a biofilm that promote the disease state. On such example is a recent study (Sandek, A. et al, Akt Ernähr Med (2006) Vol. 31) associating chronic heart failure (CHF) with a state of chronic inflammation associated with biofilm in the intestine. Given the discovery by the inventor that compositions of the present invention are effective for inhibiting biofilm reconstitution and the fact that the intestine may be treated with oral therapeutics such as syrups, liquids, lozenges, tablets, caplets, etc., the invention may provide compositions for treating biofilm-related or augmented biofilm-related conditions such as CHF by decreasing the augmented biofilm lining the intestine.

Compositions may also comprise additional ingredients, provided that they do not chemically interact with, or otherwise interfere with the action of, the active ingredients of the composition. Such ingredients can comprise, for example, additional moisturizing agents, gelling agents, lotion, cream, or other bases, antibiotic compositions, antifungal compositions, compositions for providing localized or systemic pain relief, etc. A composition could comprise MDPP and/or MDPP-A and xylitol in combination with one or more antibiotics in a moisturizing gel base. Compositions may also comprise, for example, growth factors such as platelet-derived growth factor, farnesol, furanone derivatives, and other agents that have been suggested for wound care.

Compositions may additionally include agents that have demonstrated effect in inhibiting biofilm formation, since some of those agents may also demonstrate inhibition of biofilm reconstitution. Biofilms most often comprise a mixed population of bacteria, with *Staphylococcus aureus,* coliform bacteria, *Bacteroides spp., Peptostreptococcus, Pseudomonas aeruginosa, Enterococcus pp.,* and *Streptococcus pyogenes* having been isolated from a diverse group of chronic wounds. Antibiotic administration has been associated with the development of biofilms, and biofilms are characteristically resistant to antibiotic therapy. Even such agents as sodium hypochlorite, applied at full strength to an established biofilm, may kill only 50% of the biofilm. Following removal of the slough, application of a composition aids in blocking reconstitution of the biofilm resident in the wound and may kill planktonic bacteria not yet established within the biofilm, particularly if one or more components of such a composition comprises at least one antibiotic. This type of composition may also be administered to treat an acute wound, such as a newly-formed diabetic ulcer, to promote healing and prevent the wound from establishing biofilm and becoming a chronic wound.

Compositions may be used for human wound therapy or for veterinary use. Compositions may be applied topically to one or more wounds of, for example, a dog, cat, or other mammal. Compositions may also be applied to a bite wound to protect a human from developing an ulcerated wound as the result of infection (often with biofilm fragments from the mouth of the animal).

Compositions may also include additional anti-plaque or anti-biofilm agents. Lactoferrin combined with RNAIII inhibitory peptide (RIP), for example, can have a synergistic effect and improves healing of chronic wounds. RIP has been described in United States Patent Number 6,291,431 (Balaban, et al.).

The invention may be further described by means of the following non-limiting examples.

### Examples

### Example 1

The Southwestern Regional Wound Care Center (Lubbock, Texas) provided 50 samples from patients with chronic wounds. In addition, 15 patients with acute wounds less than 24 hours in duration were biopsied and their wound beds examined. The wound samples were evaluated using Gram staining and scanning electron microscopy.

The Gram stains of the chronic wounds indicated that there were multiple species of bacteria. Also, bacteria tend to penetrate deeply into intact tissue with capillaries present, and there was quite a bit of amorphous material surrounding the bacteria. Under scanning electromicroscopy there appeared to be organized biofilm with extracellular polymeric substance adhered around colony bacteria in at least 60 percent of the chronic wounds.

The same methodology was used to evaluate 15 acute wounds. Only one of the 15 wounds was found to have biofilm. All of the acute wounds that were sampled were healed in two to three weeks, indicating no impairment to the healing process. Most of these wounds Were in the same limb and even in the same area as a chronic wound that was present. Many of these acute wounds were secondary to tape tears or trauma from the dressings being used to treat a chronic wound. Yet they healed quickly in two to three weeks, whereas the chronic wound in the same area was still open two to three months later.

In Examples 2-11, patients' wounds were treated with an anti-biofilm agent comprising bovine lactoferrin (as Bioferrin® 1000 or Bioferrin® 2000 from Glanbia Nutritionals, Inc., Monroe, Wisconsin) at 20-mg/cc concentrations. Xylitol (one-, two-, three-, four-, and five-xypentane hydro from Spectrum Chemical) comprised the second active ingredient, and was present at a concentration of 5% by weight of the mixture. The base gel of the composition comprised methylcellulose (.023 gm/cc), gelatin (.013 gm/cc) and sterile water.

### Example 2

A 62-year-old diabetic white male had developed severe peripheral neuropathy and peripheral vascular disease. He presented with the wound necrosis of his left great toe pictured in Fig. 1a with the infection tracking down the flexor tendon of his foot into the heel. He had very poor vascular status. This qualifies as a Wagner's V classification diabetic foot ulcer. The patient was very malnourished and had very difficult to manage diabetes, with blood glucose out of control (over 400) during the first 12 weeks of management.

This gentleman underwent debridement on a once weekly basis, removing necrotic tissue including bone down to healthy bleeding bone. He had IV antibiotics daily for the first eight weeks and daily dressing changes for the first four weeks and then on Monday, Wednesday, and Friday thereafter. The initial dressing changes comprised lactoferrin and a commercially available gel (Curasol® Hydrogel Wound Dressing, Healthpoint, Ltd.) along with Acticoat® (Smith & Nephew Wound Care, Hull, UK) . The patient responded well to local treatment. Because of the osteomyelitis, antibiotics were continued for a full eight weeks. As shown in Fig. 1b, the wound healed. The patient subsequently became ambulatory and had no further problems with his foot. His diabetes became much easier to control, he became better nourished and his general health improved.

### Example 3

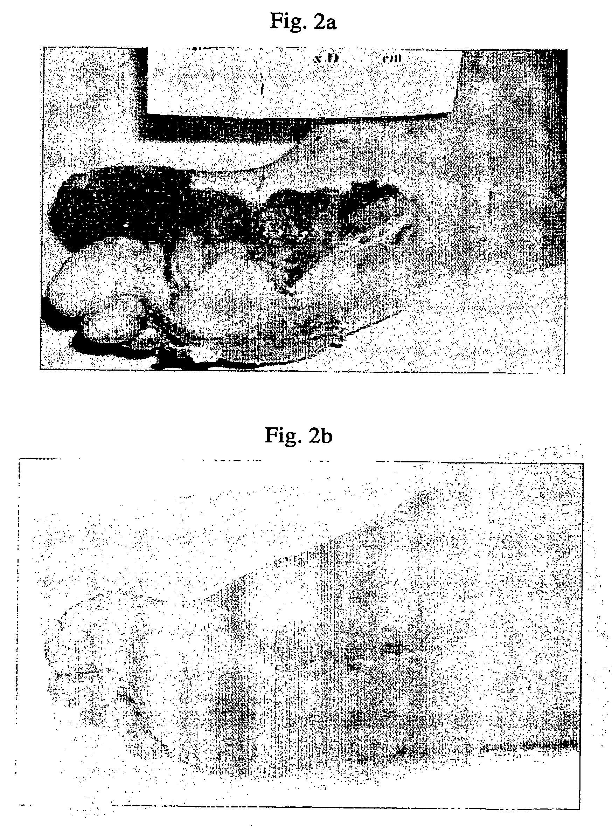

A 62-year-old Latin American male experienced diabetic foot ulcer of the left foot that began with trauma to his great toe and quickly eroded into the mid foot. The patient had extensive necrotic damage throughout the forefoot with tracking into the hindfoot consistent with a Wagner's V classification. The wound had begun with trauma, and infection was established in the great toe. Wound care, including IV antibiotics, local debridement, anti-biofilm agents and specific biocides to manage the surface, had been instituted to stop the spread of infection. Wound care was performed on a daily basis, yet the wound died back into the mid portion of the foot, as shown in Fig. 2a.

Lactoferrin therapy was instituted and the patient responded fairly quickly. Within several weeks, the progressive necrosis of the wound abated. The wound bed became granular with texture and color consistent with that of a healing wound. The drainage, pain and swelling in the left foot resolved. Seven months later the patient had complete healing of his wound, as shown in Fig. 2b. He was able to return to full activities including ambulation.

### Example 4

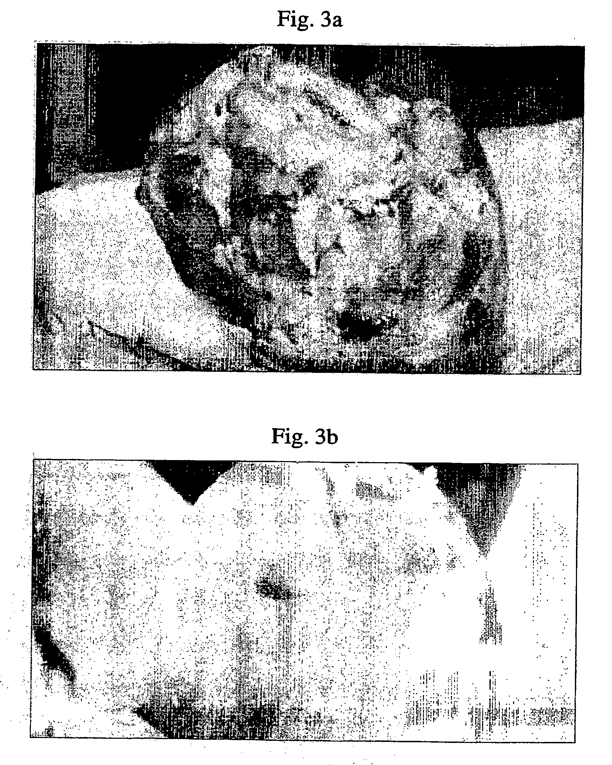

This gentleman underwent a transmetatarsal amputation of his left foot. His wound completely dehisced. The suture material was removed and a dense, fibrous slough covered the entire surface of the wound bed. The foot was swollen and tender, and the wound produced a significant quantity of exudate (Fig. 3a). The findings at the time of each debridement were consistent with a dense wound biofilm.

The patient was started on Bioferrin^{®} therapy after the sutures were out and there were 2 or 3 surface debridements. The patient responded very well to Bioferrin^{®} therapy. The drainage quickly reduced, the pain decreased and there was less swelling in the foot. The patient was continued on IV antibiotics, topical lactoferrin, specific biocides alternating between silver and Hydrofera Blue^{®} Hydrofera, LLC, Willimantic, CT) and moist interactive dressings to maintain the appropriate moisture of the surface. Within about five months, the wound had completely healed and the patient was ambulatory on the foot, returning to normal activity.

### Example 5

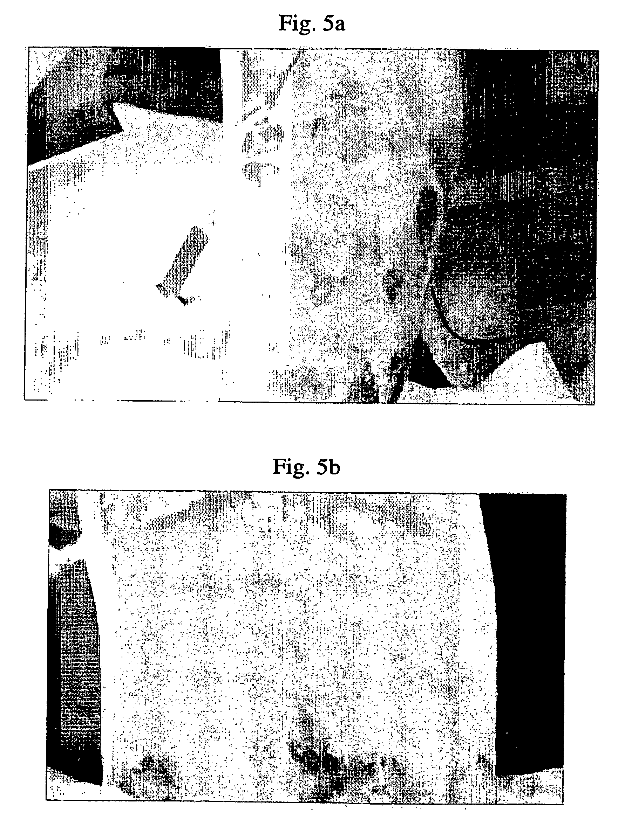

This gentleman presented to The Wound Care Center for multiple admissions over a period of 3 years prior to the photo shown in Fig. 5a. The patient had venous insufficiency in the leg, which waxed and waned. The wounds did not heal, but there were periods of quiescence when both wound drainage and pain were decreased. The patient returned with a new exacerbation of his left lower extremity pain. There was a significant amount of slough on the wound with red active borders, significant drainage and odor, and severe pain. These symptoms were recognized as indicative of the presence of active biofilm in the wound, and the patient was started on lactoferrin therapy along with compression. His primary dressings were mainly Biatain^{®} (Colplast A/S, Ltd., Humlebaek, Denmark) foam, with PolyMem^{®} (Ferris Corp., Burr Ridge, IL) used occasionally to control the moisture at an appropriate level. Over the next 5 weeks all wounds on his left lower extremity completely healed, and there has since been no regression. In this particular case, after 1 week of lactoferrin therapy the patient's wounds were very clean, with no evidence of slough (biofilm) on any of the wounds. The exudate dramatically decreased, the pain decreased, and the swelling involving his entire leg dramatically reduced over the first 2 weeks of therapy. As shown in Fig. 5b, after about six weeks, the patient's wounds were almost completely healed. He was able to return to his job and the level of function he enjoyed prior to the exacerbation.

### Example 6

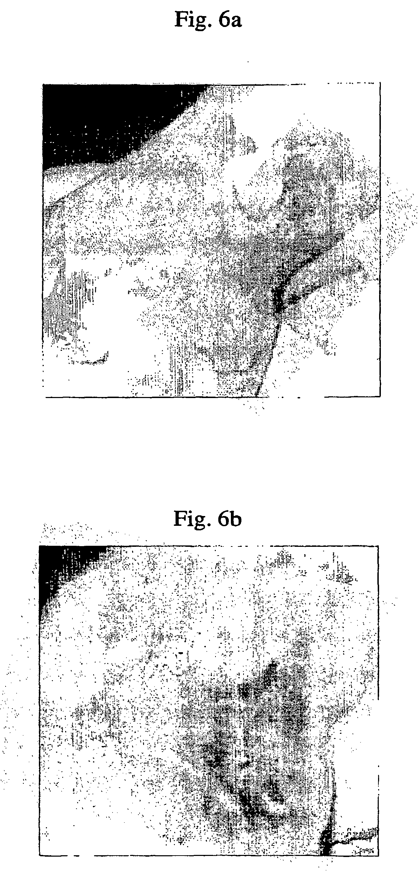

The large chronic wound seen in Fig. 6a on the right thigh of this patient exhibited characteristics of significant biofilm formation, as seen by the appearance of the slough in Fig. 6a. The wound was treated with a lactoferrin/xylitol composition of the present invention, admixed with Aquaphor® (Smith and Nephew Wound Care, Hull, UK). As shown in Fig. 6b, after a few weeks this wound, which had previously been refractory to standard treatments, was almost completely healed.

### Example 7

A 74-year-old Hispanic male underwent a right below-knee amputation. The wound dehisced and the gentleman presented with a necrotic wound shortly thereafter. Removal of the necrotic material from the wound took several weeks. The patient was treated with milk-derived protein product/xylitol gel, the milk-derived protein product comprising predominantly the apolactoferrin product Bioferrin^{®} 1000 (Glanbia Nutritionals) milk-derived protein product admixed with a Bioferrin^{®} 2000 milk-derived protein product comprising a less iron-depleted form of lactoferrin. The wound progressed rapidly to closure, although the patient was among those having multiple risk factors for chronic wound establishment-diabetes mellitus, peripheral vascular disease, malnutrition and renal failure on dialysis.

### Example 8

A 60-year-old female presented with a deep venous leg ulcer in early March. The wound exhibited signs of significant biofilm incorporation. The patient had no significant comorbidities. The wound responded well to a composition comprising Bioferrin^{®} 1000 and moved to closure within 8 weeks.

### Example 9

The patient presented with a severe venous leg ulcer of the right medial ankle which had been present for almost a year. The wound bed exhibited extensive fibrosis. The wound was debrided aggressively on a weekly basis, and a composition comprising Bioferrin^{®}1000/xylitol gel was applied to the wound. The wound progressed to healing, despite the patient's severe venous insufficiency.

### Example 10

An 82-year-old Hispanic male presented with critical limb ischemia of the right foot. He was revascularized shortly after he presented with gangrenous toes. The patient was aggressively debrided and managed primarily with a topical composition comprising Bioferrin^{®} 1000. Despite the extreme severity of the wound and the loss of tissue, the wound progressed to healing and no additional tissue removal was required (*i.e.,* the limb did not require amputation).

### Example 11

A 39-year-old diabetic Hispanic male with osteomyelitis of the left toe, significant peripheral vascular disease and a neuropathic foot was advised to have his left amputated due to a non-healing diabetic wound. He declined the amputation and presented to the Wound Care Center for limb salvage. The wound was debrided on a weekly or biweekly basis, resulting in loss of most of the bone to the great toe. The toe was salvaged, however, and the wound healed by application of a composition comprising Bioferrin® 1000 and xylitol.

### Example 12

A very pleasant 84-year-old white female presented with a large non-healing surgical wound of her chest. She had undergone aortocoronary bypass grafting with total dehiscence of her chest wound. A vacuum assisted closure had been present for almost 2 months before she presented to the Wound Care Center. The wound contained significant necrotic material at the base with 100% slough over the base. The base of the wound was irregular and there was copious drainage and a lot of erythema.

The patient was original treated with Bioferrin^{®} 2000 (lactoferrin from milk-derived protein) with xylitol. She underwent frequent debridement and anatomical management of the wound, and after 4-5 weeks still had patches of slough and necrotic material with a dull wound bed. Significant drainage was still present.

The treatment was changed to Bioferrin^{®} 1000 with xylitol approximately 5 weeks after Biofenin^{®}2000 therapy had been initiated. After about 2 ½ weeks of treatment with this composition, the wound exhibited good contraction, less necrotic material, an active edge around the wound, and was clearly healing. Within 3 months, there was a beefy red wound base, and the wound was healed a month later. The wound improved significantly when Bioferrin^{®} 1000 was introduced. The patient tolerated the treatments very well and experienced rapid closure of a very significant wound.

### Example 13

A very pleasant 58-year-old white male status post aortocoronary bypass graft presented at the Wound Care Center. He developed sternal osteomyelitis and his sternum was subsequently removed. Postoperatively his wound dehisced and he developed a large chest wound. The patient was treated with vacuum assisted closure for several months and this failed. When the inventor first saw him he had a very dusky wound bed with 100% slough covering the wound. The patient was started on Bioferrin^{®} 2000 with xylitol and did not tolerate the preparation because it caused too much burning. The patient was switched to Bioferrin^{®} 1000, which was well-tolerated and showed good response in the wound bed. A month later the entire slough was mobilized from the surface. He had active edge with contraction of the wound. Within 3 months of initial presentation, the wound showed good reduction in size and proceeded a few weeks later to complete healing.

### Example 14

A very pleasant 70-year-old female had a very difficult to heal right medial ankle ulcer infected with methicillin-resistant staph aureus (MRSA) and had been on a variety of anti-staph antibiotics including Cubicin^{®} (Cubist Pharmaceuticals, Lexington, MA), Tygacil^{®} (Wyeth Corp., Madison, NJ), Zyvox^{®} (Pfizer Caribe Ltd., Guernsey, UK) and Vancomycin. The patient's wound had been treated with Bioferrin 2000 2-3 months before Bioferrin^{®} 1000 with xylitol, combined with Acticoat, therapy was begun. By 6/16/06 the biofilm appeared to be suppressed and the patient's wound was healing rapidly. A few weeks later the wound was completely healed.

### Example 15

A very pleasant 71-year-old female had a very long history of non-healing wound of her left shin. She had venous insufficiency and associated wounds on her legs for several months. She was treated at the Wound Care Center with Bioferrin^{®} 2000 for over 5 months and then switched to Bioferrin^{®} 1000. The yellowish fibrous tissue that was in the base of the wound, which clinically appeared to be biofilm, soon disappeared and there were fairly clean wound beds on both wounds. Within a few weeks after initiation of Bioferrin^{®} 1000 therapy, the wound was healed.

### Example 16

A 46-year-old male had a venous leg ulcer on his left medial ankle or over a year and a half without showing improvement. Bioferrin^{®} 1000 therapy was instituted and in less than 6 months the wound was healed.

### Example 17

A male had venous leg ulcers continuously on the right lower extremity for many years. The size increased and decreased intermittently, but the wound never completely healed. The patient was treated with Bioferrin^{®} 2000 from October until May at which time Bioferrin^{®} 1000 therapy was initiated. The extensive area of wounding from just below the knee down to his ankle responded very quickly to the Bioferrin^{®} 1000. The slough, apparent in May within 4 weeks with just a small patch in the mid-shin region. By mid-August the shin wound was almost closed. By mid-October the wound was healed.

### Example 18

A 43-year-old male was kicked by a bull in the right lower leg, shattering his tibia and fibula and causing a compartment syndrome. A metal plate was placed on the proximal fibula. The patient was placed on Cubicin^{®} 6mg/kg with frequent irrigations and debridement of the wound and Hydrofera Blue^{®} was packed into the wound. After minimal progress, the wound appeared to be non-healing. The presence of the metal plate and screw made the wound more difficult to heal, the device providing an environment for biofilm growth. Bioferrin^{®} 1000 and xylitol was injected into the hole down the middle of the screw as an attempt to avoid hardware removal. Another 4-week course of Cubicin^{®} 6mg/kg daily was provided and the inventor continued to apply Bioferrin^{®} 1000 and xylitol into the screw on a daily basis. Although it took several more weeks, the patient's wound completely closed with no drainage and the device did not require removal but remained in place.

## Claims

1. A wound care composition comprising a combination of a milk-derived protein product comprising at least 2 percent lactoferrin by weight and xylitol for use in the treatment of biofilm-associated wounds.

2. A composition for use according to claim 1 wherein the milk-derived protein product comprises from about 2 to about 200 mg per cc of the composition.

3. A composition for use according to claim 1 wherein the milk-derived protein product is a bovine milk-derived protein product.

4. A composition for use according to claim 1 wherein xylitol comprises from about 1 percent (w/v) to about 50 percent (w/v) of the composition.

5. A composition for use according to claim 1 wherein xylitol comprises from about 5 percent (w/v) to about 20 percent (w/v) of the composition.

6. A composition for use according to claim 1 further comprising a pharmaceutically acceptable carrier.

7. A composition for use according to claim 1 wherein the pharmaceutically acceptable carrier is chosen from among the group consisting of foams, alginates, hydrocolloids, wound fillers, hydrogels and films.

8. A composition for use according to claim 1 further comprising silver.

9. A composition for use according to claim 1 wherein the lactoferrin comprises from about 0 to about 15 mg Fe/100g lactoferrin.

10. A composition for use as claimed in claim 1 wherein the lactoferrin comprises from about 15 to about 40 mg Fe/100g lactoferrin.

11. A wound care composition comprising a bovine milk-derived protein product comprising from about 50% to about 100% of the milk-derived protein product as lactoferrin and wherein the lactoferrin comprises from about 0 to about 15 mg Fe/100g lactoferrin, and at least about 5% xylitol for use in increasing the rate of healing of a biofilm-associated wound.

12. A wound dressing for biofilm-associated wounds comprising silver, a milk-derived protein product comprising at least 2 percent lactoferrin by weight or isolated lactoferrin, and xylitol.

13. A wound dressing as claimed in claim 12 wherein the silver is provided by a pharmaceutical composition.

14. A wound dressing as claimed in claim 12 wherein the silver is provided by a medical device.

## Patentansprüche

1. Zusammensetzung zur Wundversorgung, die Folgendes umfasst: eine Kombination aus einem aus Milch gewonnenen Proteinprodukt, das mindestens 2 Gewichtsprozente Lactoferrin umfasst, und Xylitol, für die Verwendung bei der Behandlung von mit Biofilm verbundenen Wunden.

2. Zusammensetzung für die Verwendung nach Anspruch 1, worin das aus Milch gewonnene Proteinprodukt von etwa 2 bis etwa 200 mg pro cm³ der Zusammensetzung umfasst.

3. Zusammensetzung für die Verwendung nach Anspruch 1, worin das aus Milch gewonnene Proteinprodukt ein aus Kuhmilch gewonnenes Proteinprodukt ist.

4. Zusammensetzung für die Verwendung nach Anspruch 1, worin Xylitol von etwa 1 Prozent (m/V) bis etwa 50 Prozent (m/V) der Zusammensetzung umfasst.

5. Zusammensetzung für die Verwendung nach Anspruch 1, worin Xylitol von etwa 5 Prozent (m/V) bis etwa 20 Prozent (m/V) der Zusammensetzung umfasst.

6. Zusammensetzung für die Verwendung nach Anspruch 1, die weiter einen pharmazeutisch verträglichen Träger umfasst.

7. Zusammensetzung für die Verwendung nach Anspruch 1, worin der pharmazeutisch verträgliche Träger aus der Gruppe ausgewählt ist, die aus Schäumen, Alginaten, Hydrokolloiden, Wundfüllstoffen, Hydrogelen und Filmen besteht.

8. Zusammensetzung für die Verwendung nach Anspruch 1, die weiter Silber umfasst.

9. Zusammensetzung für die Verwendung nach Anspruch 1, worin das Lactoferrin von etwa 0 bis etwa 15 mg Fe/100 g Lactoferrin umfasst.

10. Zusammensetzung zur Verwendung nach Anspruch 1, worin das Lactoferrin von etwa 15 bis etwa 40 mg Fe/100 g Lactoferrin umfasst.

11. Zusammensetzung zur Wundversorgung, die Folgendes umfasst: ein aus Kuhmilch gewonnenes Proteinprodukt, das von etwa 50 % bis etwa 100 % des aus Milch gewonnenen Proteinprodukts als Lactoferrin umfasst und worin das Lactoferrin von etwa 0 bis etwa 15 mg Fe/100 g Lactoferrin umfasst, und mindestens etwa 5 % Xylitol, für die Verwendung bei der Erhöhung der Heilungsrate einer mit Biofilm verbundenen Wunde.

12. Wundverband für mit Biofilm verbundenen Wunden, der Folgendes umfasst: Silber, ein aus Milch gewonnenes Proteinprodukt, das mindestens 2 Gewichtsprozente Lactoferrin umfasst, oder isoliertes Lactoferrin und Xylitol.

13. Wundverband nach Anspruch 12, worin das Silber durch eine pharmazeutische Zusammensetzung bereitgestellt wird.

14. Wundverband nach Anspruch 12, worin das Silber durch ein Medizinprodukt bereitgestellt wird.

## Revendications

1. Composition pour soins des plaies comprenant une combinaison d'un produit protéique dérivé du lait contenant au moins 2 pour cent en poids de lactoferrine et du xylitol pour une utilisation dans le traitement de plaies associées à un biofilm.

2. Composition pour l'utilisation selon la revendication 1, dans laquelle la quantité de produit protéique dérivé du lait va d'environ 2 mg à environ 200 mg par cc de la composition.

3. Composition pour l'utilisation selon la revendication 1, dans laquelle le produit protéique dérivé du lait est un produit protéique dérivé du lait de bovins.

4. Composition pour l'utilisation selon la revendication 1, dans laquelle le xylitol représente d'environ 1 pour cent (p/v) à environ 50 pour cent (p/v) de la composition.

5. Composition pour l'utilisation selon la revendication 1, dans laquelle le xylitol représente d'environ 5 pour cent (p/v) à environ 20 pour cent (p/v) de la composition.

6. Composition pour l'utilisation selon la revendication 1, qui comprend également un véhicule pharmaceutiquement acceptable.

7. Composition pour l'utilisation selon la revendication 1, dans laquelle le véhicule pharmaceutiquement acceptable est sélectionné dans le groupe consistant en des mousses, alginates, hydrocolloïdes, produits de remplissage des plaies, hydrogels et films.

8. Composition pour l'utilisation selon la revendication 1, qui comprend également de l'argent.

9. Composition pour l'utilisation selon la revendication 1, dans laquelle la lactoferrine comprend d'environ 0 à environ 15 mg de Fe/100 g de lactoferrine.

10. Composition pour l'utilisation selon la revendication 1, dans laquelle la lactoferrine comprend d'environ 15 à environ 40 mg de Fe/100 g de lactoferrine.

11. Composition pour soins des plaies comprenant une combinaison d'un produit protéique dérivé du lait de bovins comprenant d'environ 50 % à environ 100 % du produit protéique dérivé du lait sous forme de lactoferrine, la lactoferrine comprenant d'environ 0 à environ 15 mg de Fe/100 g de lactoferrine, et au moins environ 5 % de xylitol, pour une utilisation visant à accélérer la vitesse de cicatrisation d'une plaie associée à un biofilm.

12. Pansement pour plaies associées à un biofilm qui comprend de l'argent, un produit protéique dérivé du lait comprenant au moins 2 pour cent en poids de lactoferrin ou la lactoferrine isolée et du xylitol.

13. Pansement pour plaies selon la revendication 12, dans lequel l'argent est fourni par une composition pharmaceutique.

14. Pansement pour plaies selon la revendication 12, dans lequel l'argent est fourni par un dispositif médical.
